# EUROPEAN PATENT APPLICATION

(11) **EP 4 685 159 A1**
(43) Date of publication of application: **28.01.2026**
(21) Application number: 24191130.4
(22) Date of filing: 26.07.2024
(51) Int. Cl.: C07K 16/28

(54) **SINGLE DOMAIN ANTIBODIES TO CD34 SUITABLE FOR TARGETING HEMATOPOIETIC STEM CELLS**

(71) Applicant: PROVIREX Genome Editing Therapies GmbH, 22547 Hamburg (DE)
(72) Inventor: Beschorner, Niklas, 22547 Hamburg (DE); Wittner, Melanie, 22547 Hamburg (DE); Kaiser, Philipp, 72076 Tübingen (DE); Rothbauer, Ulrich, 72072 Tübingen (DE)
(74) Representative: Moré, Solveig Helga

(57) **Abstract**

The present invention relates to single domain antibodies - also designated nanobodies - to CD34 as well as nucleic acids encoding them and pharmaceutical compositions comprising them. Specific nanobodies capable of binding to human CD34 on cells, e.g., on hematopoietic stem cells, are provided. These are useful for targeting agents, e.g., gene transfer moieties such as lipid nanoparticles (LNP), to the cells in vitro or in vivo, for example in the context of genetic engineering of CD34+ cells. The nanobodies are also useful for diagnostic or for imaging applications.

## Description

The present invention relates to single domain antibodies - also designated nanobodies - to CD34 as well as nucleic acids encoding them and pharmaceutical compositions comprising them. Specific nanobodies capable of binding to human CD34 on cells, e.g., on hematopoietic stem cells, are provided. These are useful for targeting agents, e.g., gene transfer moieties such as lipid nanoparticles (LNP), to the cells in vitro or in vivo, for example in the context of genetic engineering of CD34+ cells. The nanobodies are also useful for diagnostic or for imaging applications.

CD34 is a transmembrane phosphoglycoprotein protein encoded by the CD34 gene in humans, mice, rats and other species. CD34 was first described on hematopoietic stem cells as a cell surface glycoprotein. It functions as a cell-cell adhesion factor. It may also mediate the attachment of hematopoietic stem cells to bone marrow extracellular matrix or directly to stromal cells. Clinically, it is associated with the selection and enrichment of hematopoietic stem cells for bone marrow transplants. CD34 is also expressed by other cells, e.g., on endothelial cells, endothelial progenitor cells, mast cells, and a subpopulation of dendritic cells. The presence of CD34 on non-hematopoietic cells in various tissues has been linked to progenitor and adult stem cell phenotypes.

CD34 is often used clinically to quantify the number of haematopoietic stem cells for use in hematopoietic stem cell transplantation. CD34+ cells may be isolated from blood samples using immuno-magnetic techniques and e.g. used for CD34+ transplants, which have lower rates of graft-versus-host disease. Cells observed as CD34+ and CD38- are of an undifferentiated, primitive form; i.e., they are multipotent hematopoietic stem cells. Thus, because of their CD34+ expression, such undifferentiated cells can be sorted. Injection of CD34+ hematopoietic stem cells has been clinically applied to treat various diseases including spinal cord injury, liver cirrhosis and peripheral vascular disease.

In tumors, CD34 is expressed by alveolar soft part sarcoma, preB-ALL (positive in 75%), AML (40%), AML-M7 (most), dermatofibrosarcoma protuberans, gastrointestinal stromal tumors, giant cell fibroblastoma, granulocytic sarcoma, Kaposi's sarcoma, liposarcoma, malignant fibrous histiocytoma, malignant peripheral nerve sheath tumors, meningeal hemangiopericytomas, meningiomas, neurofibromas, schwannomas, and papillary thyroid carcinoma. A negative CD34 may exclude Ewing's sarcoma/PNET, breast myofibrosarcoma and inflammatory myofibroblastic tumors of the stomach.

Antibodies are typically used to quantify and purify hematopoietic stem cells for research and applications such as for clinical bone marrow transplantation. However, antibodies are large proteins which can be costly to produce. Antibodies can also be used for targeting CD34+ cells *in vivo,* but classical antibodies are not ideal for such tasks, e.g., due to their size. Single domain antibodies or nanobodies which due to their small size can be more easily produced and which can better infiltrate tissues are known in the art, however, so far, no nanobodies to CD34 have been available.

In light of this, and to overcome at least some of the disadvantages associated with classical antibodies, the inventors have addressed the problem of providing single domain antibodies to CD34, in particular, human CD34. The problem has been solved by the present invention as described herein, in particular, by the subject matter of the claims.

The invention provides a nucleic acid encoding a single domain antibody (also designated nanobody) capable of specifically binding to human CD34, the single domain antibody comprising a CDR1 having at least 60%, preferably, at least 80% sequence identity to SEQ ID NO: 1, a CDR2 having at least 80%, preferably, at least 90% sequence identity to SEQ ID NO: 2, and a CDR3 having at least 85% sequence identity to SEQ ID NO: 7.

Further single domain antibodies were produced, comprising:
i. a CDR1 having at SEQ ID NO: 20, a CDR2 having SEQ ID NO: 21, and a CDR3 having SEQ ID NO: 22,
ii. a CDR1 having at SEQ ID NO: 23, a CDR2 having SEQ ID NO: 24, and a CDR3 having SEQ ID NO: 25,
iii. a CDR1 having at SEQ ID NO: 26, a CDR2 having SEQ ID NO: 27, and a CDR3 having SEQ ID NO: 28,
iv. a CDR1 having at SEQ ID NO: 29, a CDR2 having SEQ ID NO: 30, and a CDR3 having SEQ ID NO: 31,
v. a CDR1 having at SEQ ID NO: 32, a CDR2 having SEQ ID NO: 33, and a CDR3 having SEQ ID NO: 34, or
vi. a CDR1 having at SEQ ID NO: 35, a CDR2 having SEQ ID NO: 36, and a CDR3 having SEQ ID NO: 34.

All of the encoded single domain antibodies are capable of binding to human CD34 protein with good specificity and affinity, comparable to classic, e.g., IgG antibodies to CD34 that are commercially available. They can e.g., be used for detecting purified folded CD34, or for detecting CD34 in Western Blots and/or FACS.

CD34, in the context of the present invention, is preferably human CD34. Hematopoietic progenitor cells of, e.g., mouse or rat also express CD34, so the CD34 can be mouse or rat CD34, but human CD34 is preferred.

A single domain antibody (sdAb), also called nanobody, is an antibody fragment consisting of a single monomeric variable antibody domain, which is able to selectively bind to a specific antigen. As single domain antibodies, the nanobodies of the invention represent an excellent alternative to conventional antibodies (e.g., IgG): With a molecular weight of only 12-15 kDa, single-domain antibodies are much smaller than common antibodies (150-160 kDa) which are composed of two heavy protein chains and two light chains, and even smaller than Fab fragments (~50 kDa, one light chain and half a heavy chain) and single-chain variable fragments (~25 kDa, two variable domains, one from a light and one from a heavy chain). In particular, the single domain antibodies of the invention can be derived from antibodies from camelids, such as llamas, camels, dromedaries or alpacas. Camelids have a unique subset of immunoglobulins consisting of heavy chain homodimers devoid of light chains. Their variable region (VHH) is the smallest antigen-binding fragment found in the antibody world, and as a single polypeptide chain it is especially suitable for protein engineering. Single domain antibodies, or "nanobodies", can be the recombinant minimal-sized, intact antigen-binding domains derived from the VHH region of these heavy-chain antibodies. Alternatively, single domain antibodies can be derived from antibodies of cartilaginous fish, such as shark, which have heavy chain antibodies from which single domain antibodies called VNAR fragments can be obtained.

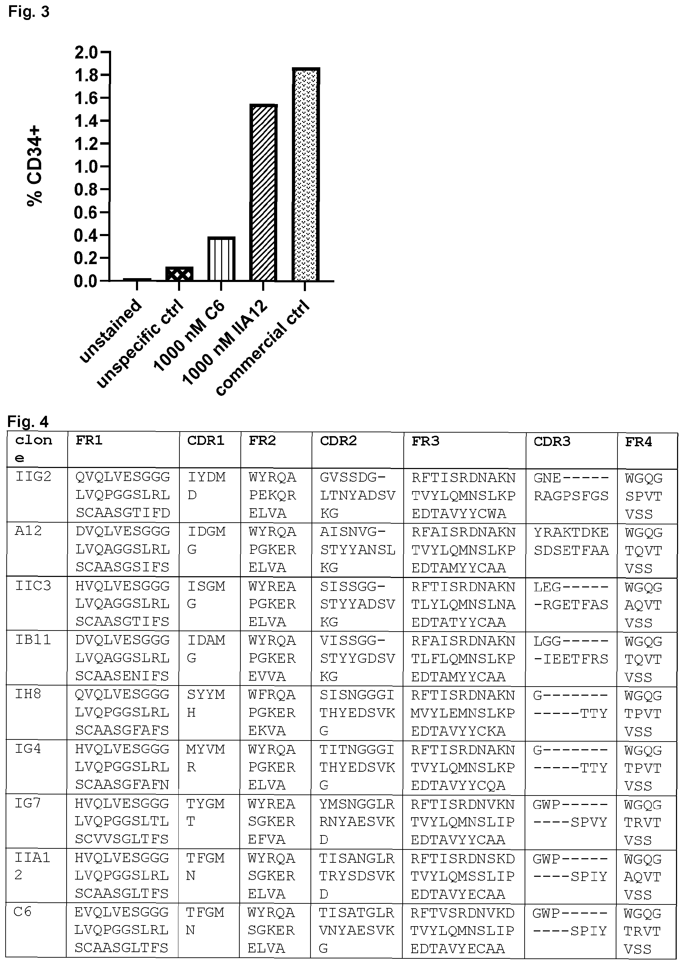

Preferably, the single domain antibodies of the invention have, in addition to the CDR regions as claimed, VHH framework regions derived from a camelid, e.g., an alpaca. Llama, camel or dromedary framework regions can also be used. Preferred framework regions are those of the respective antibodies from which the CDRs were identified, as described, e.g., in Fig. 4, however, other suitable framework regions can also be employed, e.g., framework regions from one of the other identified antibodies, or combinations thereof. In the context of the invention, a CDR having a sequence consists of said sequence. The total number of amino acids in the single domain antibody may be, e.g., in the range of 113-124, e.g., 116.

Unlike monoclonal antibodies, single domain antibodies can be readily produced in large amounts in simple bacterial expression systems. Moreover, nanobodies are usually extremely stable, can bind antigens with affinities in the nanomolar range, and are smaller in size and thereby easier to manipulate genetically as compared with antibody fragments such as single chain variable fragments.

Also, due to the small size and compact folding nanobodies show a high chemical stability, solubility and fast tissue penetration. Additionally, nanobodies can be easily converted into multivalent formats, such as bivalent, trivalent, tetravalent or other multi-valent, optionally, multi-specific or biparatopic constructs, e.g. addressing different epitopes on the same antigen. The nanobodies of the invention can also be part of such constructs, e.g., two or more nanobodies of the invention may be linked together. Multivalency can increase avidity.

Also, nanobodies have comparable antigen specificities and affinities to classical immunoglobulins. Due to their high homology with human antibody (VH) fragments, show only very low immunogenicity. Further, single domain antibodies can optionally be humanized to further reduce immunogenicity. Most of the antibodies generated for the present application, e.g., those having the CDRs as specified above under i-vi do not bind to CD34 expressed on cells. It is surprising and particularly advantageous that a group of nanobodies is provided by the invention that is able to target human CD34 in the cellular context and can thus not only be used for detecting the protein, but also to detect CD34-expressing cells or to target moieties to such cells: Therefore, the invention provides a nucleic acid encoding a single domain antibody (also designated nanobody) capable of specifically binding to human CD34, the single domain antibody comprising
a) a CDR1 having SEQ ID NO: 1, a CDR2 having SEQ ID NO: 2, and a CDR3 having SEQ ID NO: 7 or
b) a CDR1 having SEQ ID NO: 17, a CDR2 having SEQ ID NO: 18, and a CDR3 having SEQ ID NO: 19.

Preferably, the CDR1 has SEQ ID NO: 1, the CDR2 has SEQ ID NO: 2, and the CDR3 has SEQ ID NO: 7. In the context of the invention, two antibodies with a CDR1 having SEQ ID NO: 1, a CDR2 having SEQ ID NO: 2, and a CDR3 having SEQ ID NO: 7 were found to have the best binding to CD34-expressing human cells. Therefore, these antibodies are particularly preferred. They have identical CDR1 and CDR3 regions, but the CDR2 differs in six positions. SEQ ID NO: 2, TISAXGLRXXYXXSVXX, is a consensus sequence of the CDR2 of these two preferred single domain antibodies, and takes this variability into account. SEQ ID NO: 2 comprises seven variable positions, as it further takes a possible substitution of a lysine in the second last (C-terminal) position of the CDR2 sequence into account, e.g., to an arginine, which can be helpful. The CDR2 sequence may also be the CDR2 consensus sequence of C6 and IIA 12 that maintains the K is said position, i.e., SEQ ID NO: 74 ofTISAXGLRXXYXXSVKX. In SEQ ID NO: 2 or 74, all variable positions X can, independently from each other, be any naturally occurring amino acid. The second last (C-terminal) position of the CDR2 sequence preferably is K or R.

More preferably, the variable amino acids are selected from specific subgroups, so that they are conservative substitutions, i.e., from the same group of amino acids with common physico-chemical properties as one of the original amino acids. Thus, the nanobodies preferably comprise SEQ ID NO: 3, TISAX1GLRX2X3YX4X5SVKX6, wherein, independently, X1 is a polar amino acid, X2 is a polar or unpolar amino acid, X3 is a basic or polar amino acid, X4 is a polar or unpolar amino acid, X5 is an acidic amino acid and X6 is an acidic or an unpolar amino acid. Polar amino acids are Y, T, Q, G, S, C or N. Unpolar amino acids are A, V, M, L, I, P, Wand F. Basic amino acids are K, R and H. Acidic amino acids are E or D.

In a preferred embodiment, the nanobodies comprise SEQ ID NO: 4, TISAX1GLRX2X3YX4X5SVKX6, wherein, independently, X1 is N or T, X2 is T or V, X3 is R or N, X4 is S or A, X5 is D or E, and X6 is D or G. These are the amino acids present in the two preferred nanobodies of the invention.

Excellent binding specificity and affinity to cells expressing human CD34 is also shown by a single domain antibody comprising a CDR1 having SEQ ID NO: 1, a CDR2 having SEQ ID NO: 6 and a CDR3 having SEQ ID NO: 7. Such a single domain antibody may have at least 90%, optionally, 100% sequence identity to SEQ ID NO: 9. A single domain antibody comprising SEQ ID NO: 9 is designated IIA12, which is the preferred single domain antibody of the invention.

A good binding specificity and affinity to cells expressing human CD34 is also shown by a single domain antibody comprising a CDR1 having SEQ ID NO: 1, a CDR2 having SEQ ID NO: 5 and a CDR3 having SEQ ID NO: 7. Such a single domain antibody may have at least 90%, optionally, 100% sequence identity to SEQ ID NO: 8. A single domain antibody comprising SEQ ID NO: 8 is designated C6.

In one embodiment, the single domain antibody of the invention does not contain any lysine residues. This makes alternative coupling method possible, which may allow for easier preparation e.g., of single domain antibody-coupled lipid nanoparticles. The CDR2 of the single domain antibody may thus have one of the consensus sequences of SEQ ID NO: 75 or, preferably, SEQ ID NO: 76. The CDR2 may be the CDR2 of C6 allowing for K substitution (SEQ ID NO: 77), wherein optionally, the K is substituted to R. The CDR2 may be alternatively be the CDR2 of IIA12 allowing for K to R substitution, wherein optionally, the K is substituted to R (SEQID NO: 78). Preferably, said single domain antibody is a variant of the single domain antibody having SEQ ID No. 8 or 9, wherein all lysine residues have been exchanged for arginine. Thus, the single domain antibody may comprise SEQ ID NO: 79, i.e., C6 with all K substituted to R. The single domain antibody may in one embodiment comprise SEQ ID NO: 80, i.e., IIA12 with all K substituted to R.

A single domain antibody that has a CDR1 having SEQ ID NO: 17, a CDR2 having SEQ ID NO: 18, or a variant thereof wherein K is exchanged for another amino acid, preferably for R, and a CDR3 having SEQ ID NO: 19 was also identified by the inventors. It can have, e.g., at least 90%, optionally, 100% sequence identity to SEQ ID NO: 10. A single domain antibody having SEQ ID NO: 10 is designated IG7. It can also bind to human CD34 expressed by cells, however, with lower affinity than the nanobodies derived from C6 or IIA7 described herein. In the single domain antibody wherein the CDR2 is a variant of SEQ ID NO: 18 in which K is exchanged for another amino acid, preferably for R, preferably, the same substitution is included for all K residues, e.g., it may be a variant of SEQ ID NO: 10 wherein all K are exchanged for R.

The nucleic acid of the invention, encoding the single domain antibody of the invention, can be any nucleic acid, e.g., in the form of a plasmid, a phage, virus, transposon, minicircle, cosmid, or chromosome. It can be a cloning construct. The nucleic acid encoding the single domain antibody is preferably linked to a heterologous sequence. Preferably, it is an expression construct wherein the sequence encoding the single domain antibody is functionally linked to a promotor, preferably, a heterologous promotor. As production of functional nanobodies does not require glycosylation, they can be expressed in all sorts of cells, e.g., bacterial cells, eukaryotic cells such as mammalian, e.g., human cells, in insect cells or in yeast cells. The promotor can be a promotor suitable for expression in any of these cells. It may be an inducible promotor or a promotor for constitutive expression. The nucleic acid may further comprise an enhancer. A bacterial expression construct can be, e.g., a plasmid or a minicircle. The nucleic acid can also be a viral vector suitable for packaging into a virus particle.

Optionally, the sequence linked to the single domain antibody may be further linked to a coding sequence encoding a fusion partner configured so that the single domain antibody is expressed as a fusion protein with the fusion partner. The fusion partner is typically heterologous. The fusion partner can be N-terminal or C-terminal to the single domain antibody in the expressed protein, as long as the binding affinity of the single domain antibody to CD34 is not significantly affected. Exemplary fusions partners are the Fc region of a human or murine immunoglobulin, e.g., IgG, an albumin-binding sequence, albumin, PAS polypeptides, HAP polypeptides, the C-terminal peptide of the beta subunit of chorionic gonadotropin, albumin-binding small molecules, streptavidin or avidin, or tags, e.g., a His-tag or FLAG-tag. To allow for targeting viral or pseudoviral particles to CD34-expressing cells, the fusion partner can also be a viral capsid protein, e.g., VP2 or VP1 of AAV, preferably, VP1. The single domain antibody is fused to the viral capsid protein such that it is presented on the outside of the viral particle and accessible to CD34, e.g. at the N-terminus of the capsid protein. For example, the single domain antibody can be fused at the N-terminus of VP2 or at in the GH2/GH3 surface loop of VP1. The single domain antibody can also be fused at least to a transmembrane region and at least one intracytoplasmic signalling domain to form a chimeric antigen receptor (CAR), optionally, via a spacer. Fusion partners can, e.g., increase stability and/or half-life of the single domain antibody, e.g., after administration to a human. They can endow the single domain antibody with an effector function, lead to dimerisation or multimerization, can simplify purification, allow for detection, and/or lead to a desired localization of the single domain antibody e.g. after *in vivo* administration. A fusion protein can also comprise several fusion partners and/or a linker, e.g., a Ser/Gly linker such as GlyGlySer.

The present invention also provides a single domain antibody encoded by the nucleic acid of the invention. Said single domain antibody may e.g., be used for detecting CD34 protein in a Western Blot. Direct labelling of the single domain antibody is not required, as secondary anti-VHH antibodies may be used for detection.

However, in a preferred embodiment, the single domain antibody of the invention is associated with a moiety selected from the group comprising
a) a transfer moiety selected from the group comprising a lipid nanoparticle (LNP), a viral particle, a liposome, an exosome or a bacterial minicell,
b) a drug
c) a detectable label selected from the group comprising a fluorophore, a radionuclide, a magnetic particle or a nucleic acid and/or
d) a moiety increasing stability and/or serum half-life such as polyethylenglycol (PEG) or hydroxyethyl starch (HES),

The moiety can be directly associated, in particular, by covalent linkage, or indirectly associated with the single domain antibody, e.g., by linkage of the single domain antibody to biotin-avidin or biotin-streptavidin interaction. The moiety can be linked via a spacer, e.g., to reduce potential steric hindrance for the binding of the single domain antibody.

Preferably, the single domain antibody is associated with a transfer moiety, selected from the group comprising a lipid nanoparticle (LNP), a viral particle, a liposome, an exosome or a bacterial minicell. An LNP comprising a single domain antibody of the invention is a preferred embodiment. A lipid nanoparticle is typically spherical with an average diameter between 10 and 1000 nanometers. Solid lipid nanoparticles (SLN) possess a solid lipid core matrix that can solubilize lipophilic molecules. The lipid core is stabilized by surfactants (emulsifiers). The emulsifier used depends on administration routes. The term lipid is used here in a broader sense and includes triglycerides (e.g. tristearin), diglycerides (e.g. glycerol bahenate), monoglycerides (e.g. glycerol monostearate), fatty acids (e.g. stearic acid), steroids (e.g. cholesterol), and waxes (e.g. cetyl palmitate). An SLN is generally spherical in shape and comprises of a solid lipid core stabilized by a surfactant. The core lipids can be fatty acids, acylglycerols, waxes, and mixtures of these surfactants. Biological membrane lipids such as phospholipids, sphingomyelins, bile salts (sodium taurocholate), and sterols (cholesterol) may be utilized as stabilizers. Biological lipids having minimum carrier cytotoxicity and the solid state of the lipid permit better controlled drug release due to increased mass transfer resistance (Wikipedia). In contrast to SLN, liposomes have a phospholipid bilayer as a coat, and the bulk of the interior of the particle is composed of aqueous substance. Exosomes, ranging in size from 30 to 150 nanometers, are membrane-coated extracellular vesicles produced in the endosomal compartment of an eukaryotic cell.

A viral particle may be a DNA virus or an RNA virus, e.g., a lentiviral particle. It may also be an adenoviral particle or an Adenovirus-associated Virus (AAV) particle, e.g. an AAV2, AAV3, AAV4, AAV5, AAV6, AAV7, AAV8, or an AAV9 particle.

A transfer moiety comprises a cargo that can be transferred, typically, an active agent such as a nucleic acid, a drug or a detectable label. In a preferred embodiment, the transfer moiety comprises a nucleic acid such as mRNA. The transfer moiety may, together with the nanoparticle, allow for transfer of the cargo, e.g., the active agent, to the cytoplasm of a targeted cell (i.e., a cell expressing CD34). For example, a nucleic acid may be transferred to a CD34-expressing cell, thus allowing for expression of said nucleic acid in the cell. The nucleic acid may be DNA or RNA, preferably, RNA. Use of a single domain antibody of the invention linked to a gene transfer moiety allows to introduce, e.g., a therapeutically relevant nucleic acid sequence of interest directly into the targeted cells in a patient, i.e., for *in vivo* gene therapy. It also allows for transfer into cells isolated from a patient or a donor, i.e., ex *vivo* gene therapy. The cells are preferably CD34+ hematopoietic stem cells. The active agent can also be a complex of a guide RNA (preferably, a sgRNA) and a Cas protein, typically, Cas9. A nucleic acid encoding the same can also be used. DNA encoding a transgene that is to be integrated into the cell can further be present in this embodiment.

Expression of therapeutic transgenes by transduced cells after gene therapy can be transient (e.g., hours to a week) or sustained (e.g., more than a week to months or longer), depending upon the specific construct used and the target tissue or cell type. Expression of the transgene can also be inducible, depending on the promotor used for the transgene. The transgene can be introduced, e.g., as an integrating or non-integrating linear construct, a circular plasmid or a viral vector. The transgene can also be introduced by virus-like particles (VLP) or chemical nanoparticle carriers (e.g. lipid nanoparticles). mRNA is a particularly suitable nucleic acid if transient expression is desired.

Examples of a transgene that may be encoded on the nucleic acid are, e.g., a fluorescent protein that enables detection of cells (e.g., GFP, CFP), a toxin, cytokine or enzyme. One transgene may be encoded, but of course, a nucleic acid may also encode a plurality of transgenes, e.g., two or three.

In one embodiment, the transgene may be an enzyme tailored to excise HIV provirus from cells, e.g. BREC, as disclosed by WO 2016/034553 A1. For example, a nucleic acid, e.g., DNA, encoding BREC may be provided in a gene transfer moiety such as an LNP associated with a CD34 single domain antibody as disclosed here, preferably, a single domain antibody having a CDR1 having SEQ ID NO: 1, a CDR2 having SEQ ID NO: 2, e.g., SEQ ID NO: 74, and a CDR3 having SEQ ID NO: 7. The single domain antibody can, e.g., be IIA12. It can also be a variant thereof wherein K is substituted to R (e.g., SEQ ID NO: 80). An LNP associated with a C6 CD34 single domain antibody and comprising a nucleic acid, e.g., mRNA, encoding BREC is also disclosed.

Transfer of the active agent to the cytoplasm is however not required. Thus, it can be sufficient if an active agent such as a drug, e.g., a toxin, or a dye is transferred to the immediate vicinity of a CD34-expressing cell, e.g., for killing that cell, for marking it for attack, e.g., by the immune system, or for detecting the cell.

A drug may be any therapeutically active agent that effects a therapeutic, i.e. beneficial treatment effect in a patient in need of such treatment. Non-limiting examples of drugs include, for example, a cytokine inhibitor, a growth factor inhibitor, an immunosuppressant, an anti-inflammatory agent, a metabolic inhibitor, an enzyme, an enzyme inhibitor, a cytotoxic agent, and a cytostatic agent. For example, if a patient has a CD34+ cancer, a drug targeted to a cancer cell as described herein can be a cytotoxic agent. The drug can be comprised in a transfer moiety, or it can be directly or indirectly linked to the single domain antibody of the invention.

Similarly, a detectable label can be comprised in a transfer moiety, or it can be directly or indirectly linked to the single domain antibody of the invention. It can also be incorporated into the single domain antibody, e.g., by replacement of an amino acid residue of the single domain antibody with a modified amino acid residue such that it becomes detectable (e.g., by radiolabeling). Non-limiting examples of detectable labels as known in the art include fluorescent labels (such as fluorescein isothiocyanate (FITC), rhodamine, lanthanide phosphors), chemiluminescent markers, enzymatic labels (such as horseradish peroxidase, beta-galactosidase, luciferase, alkaline phosphatase), nucleic acids and radioisotopes or radionuclides (such as ³⁵S, ¹¹C, ¹³N, ¹⁵O, ¹⁸F, ¹⁹F, ⁹⁹TC, ¹³¹I, ³H, ¹⁴C, ¹⁵N, ⁹⁰Y, ⁹⁹Tc, ¹¹¹In and ¹²⁵I). The label may be directly detectable or may be detectable only after contact with further compounds compositions or molecules, e.g., in the case of predetermined polypeptide epitopes recognized by a secondary reporter (such as a leucine zipper pair sequences, binding sites for secondary antibodies, metal binding domains, or epitope tags). In one example, the label may be the incorporation of a radiolabeled amino acid, which is directly detectable according to methods known in the art. In additional or alternative examples, the label may be the attachment of biotin moieties to the single domain antibody, which are detectable following contact with marked avidin or streptavidin. Magnetic agents (such as gadolinium chelates) are another example of detectable labels. Often, the detectable label is also selectable, e.g., in the case of fluorescent or magnetic labels or nucleic acid labels. In one embodiment, the detectable label is a magnetic bead allowing for selection of CD34-positive cells by magnetic cell sorting.

According to one aspect of the invention, the detectable label is selected from detectable moieties and tracers for immuno-histochemistry, optical imaging, near infrared imaging (NIR), positron emission tomography (PET), single photon emission computed tomography (SPECT) or magnetic resonance imaging (MRI).

In order to label a single domain antibody of the invention, any method known in the field can be applied. Generally, to label the single domain antibody of the invention for, e.g., positron emission tomography (PET), PET tracers are coupled to a chelator (e.g. NODAGA) or click-reagent via a reactive unit (e.g. primary amino group, azide group in combination with DBCO, C-terminal cysteine, His6-tag) located within the single domain antibody, via which the radionuclide is then introduced.

The invention also provides a host cell comprising the nucleic acid of the invention and preferably, expressing the single domain antibody of the invention. The host cell can be, e.g., a bacterial cell, e.g., E. *coli,* a yeast cell, e.g. S. *cerevisiae* or S. *pombe,* an insect cell or a mammalian cell, e.g., a BHK cell or a HEK cell or a CHO cell.

Another object of the invention is to provide a pharmaceutical composition comprising the nucleic acid of the invention, the single domain antibody of the invention or the host cell of the invention expressing said single domain antibody. In the context of a pharmaceutical composition of the invention, the single domain antibody comprises a CDR1 having at least 80% sequence identity to SEQ ID NO: 1, a CDR2 having at least 90% sequence identity to SEQ ID NO: 2, and a CDR3 having at least 85% sequence identity to SEQ ID NO: 7. For example, the CDR2 can have SEQ ID NO: 74. Accordingly, the pharmaceutical composition comprises a single domain antibody of the invention that is capable of binding to human CD34 expressed by a cell. This also applies for all medical, therapeutic, diagnostic or cell detection uses described herein.

Optionally, the pharmaceutical composition further comprises at least one pharmaceutically acceptable excipient and/or solvent, e.g., water, physiological saline or a buffer such as PBS (phosphate buffered saline). A preferred buffer is TRIS. The pharmaceutical composition may, e.g., comprise a dispersing or suspending agent, surfactant, isotonic agent, spreader or emulsifier, encapsulating agent, lyoprotectant, bulking agent, stabilizer or preservative. An overview of such pharmaceutically acceptable ingredients can be found in, for example, Rowe (Ed.) *et al.:* Handbook of Pharmaceutical Excipients, 7th edition, 2012, Pharmaceutical Press. A preferred excipient is a sugar such as trehalose or sucrose. As the subject or patient whom the composition is to be administered is a human, it has to be safe for human use. In a preferred embodiment, the pharmaceutical composition comprises TRIS, e.g., at 10 mM and sucrose, e.g., 9%, in particular, if the single domain antibody is associated with an LNP.

Preferably, the pharmaceutical composition is a liquid composition. It may be stored frozen, e.g., to increase stability. It may also be a dry composition, e.g., a lyophilized composition that may be more stable and that may, e.g., be dissolved before administration.

The pharmaceutical composition can be for use in administration by a parenteral route, e.g., by subcutaneous, intravenous, intramuscular or intraperitoneal injection, or by oral or topical administration, by intravaginal or rectal administration or by inhalation. Preferably, it is for use in intravenous application.

The invention further provides use of the single domain antibody of the invention comprising a single domain antibody associated with a moiety for targeting the moiety to a cell expressing human CD34, preferably *in vitro.* Said cell optionally is a human hematopoietic stem cell. For example, the cell may have been obtained from a human subject, then it may be contacted with the single domain antibody of the invention associated with the moiety, and it may then be for use in administration to a human subject, preferably, the same human subject from which it has been obtained. During the in vitro treatment, preferably, the CD34-expressing cells are transfected with a nucleic acid. Then, the transfected cells can be (re-) administered to the subject. This enables an ex vivo gene therapy. For example, human hematopoietic cells can be mobilized, isolated and concentrated from peripheral blood and then contacted with the nanobodies under suitable conditions to allow transfer of a nucleic acid into the cells, e.g., with LNP associated with the nanobodies comprising DNA or mRNA or a protein/RNA complex, e.g., cas (typically, cas9) associated with a gRNA. For example, a nucleic acid, e.g., mRNA, encoding a recombinase can be transferred to CD34+ hematopoietic stem cells, e.g., in to repair a gene defect.

For mobilization, stem cells are stimulated out of the bone marrow space into the bloodstream in order to increase the number of stem cells in the peripheral blood prior to collection from the individual. Two common methods of mobilization that may be used in the context of the invention are administration of colony stimulating factors (CSFs) and chemotherapy, e.g., as described in Hopman RK, DiPersio JF. Advances in stem cell mobilization. Blood Rev. 2014 Jan;28(1):31-40. doi: 10.1016/j.blre.2014.01.001. Isolation of mobilized CD34-expressing cells from peripheral blood mononuclear cells can e.g. be performed by using magnetically labelled anti-CD34 antibodies, which bind to the target cells and then to an external magnet which allows for decanting of any unwanted cells while the cells of interest remain in the reagent tube. The isolation process also concentrates the stem cells.

The pharmaceutical composition of the invention comprising a single domain antibody associated with a moiety can also be for use in targeting the moiety to a cell expressing human CD34, such as a human hematopoietic stem cell. The pharmaceutical composition is to be administered to a human subject in need thereof. In a preferred embodiment, it is for use in transfecting a cell expressing human CD34 with a nucleic acid, wherein the moiety is a transfer moiety comprising a nucleic acid, e.g., a nucleic acid encoding a recombinase, a TALEN or ZNF. The nucleic acid may e.g. be RNA in this context. The transfer moiety may alternatively comprise a Cas/gRNA complex or a nucleic acid encoding the same. This allows for *in vivo* gene therapy of cells having a gene defect. Alternatively, if the subject has a cancer with CD34+ cancer cells, the single domain antibody of the invention may be associated with a drug. It may then be used to target the associated drug, e.g., a toxin or a cytostatic drug to the cancer cells. The single domain antibody may also be comprised in a CAR, and T cells comprising said CAR may be for use in treatment of a subject having a CD34+ cancer.

The invention also provides a method of treating a human subject in need thereof, comprising administering the pharmaceutical composition to the patient. The subject can e.g., be a subject having a gene defect or a cancer patient having a CD34+ cancer.

Gene defects that can be addressed for treatment and/or prevention of the associated diseases are present, e.g., in sickle cell disease, thalassemia, ADA deficiency, chronic granulomatous disease, haemophilia A, Fanconi anemia or pyruvate kinase deficiency.

Further, the invention provides a method of preparing a genetically engineered human cell expressing CD34 (i.e., the cell that is to be genetically engineered expresses CD34), the method comprising
a) *in vitro* contacting a cell expressing human CD34 with a single domain antibody of the invention, or
b) administering to a human subject in need thereof a pharmaceutical composition of the invention,
wherein the moiety is a transfer moiety comprising a nucleic acid. The method optionally is a method for preparing a genetically engineered human hematopoietic stem cell. Said nucleic acid typically encodes a transgene, and the genetically engineered cell expresses the transgene or is capable of expressing it. The transgene may be stably or transiently expressed, depending on the nucleic acid and promotor. For example, if the transgene is operably linked to an inducible promotor, expression can be inducible. The promotor is active in human hematopoietic stem cells or other human CD34+ cells. The nucleic acid may alternatively be a gRNA associated with Cas, typically, Cas9, for correction of a gene defect in the target cell.

Further, the invention provides a method for detecting a cell expressing human CD34, the method comprising
a) *in vitro* contacting a human cell expressing CD34 with a single domain antibody of the invention, wherein the cell is optionally comprised in a mixture of different cells (e.g., in PBMC) or in a tissue or organ, e.g., in a biological sample taken from a subject, or
b) administering to a human subject a single domain antibody of the invention, and determining binding said single domain antibody to a cell.

A single domain antibody of the invention associated with a detectable label can be used for different approaches for imaging cells. Alternatively, a secondary antibody associated with a detectable label, e.g., a VHH antibody may be used. Detection of binding can be by various means, e.g., known in the art. *In vitro,* binding can for example be determined by flow cytometry or an ELISPOT assay. *In vivo,* various imaging approaches can be used. Noninvasive imaging approaches offer a significant benefit compared to current diagnostic standard. Nanobodies, e.g., associated with a contrast agent have significant advantages to conventional antibodies in this context. Due to the recycling effect mediated by the neonatal Fc receptor, full-length antibodies have a long serum half-life (~ 1-3 weeks), which requires long clearance times (several days) before high-contrast images can be acquired. Additionally, effector function via the Fc region may induce depletion or functional changes in targeted cells including the induction of proliferation or cytokine release. With the nanobodies presented herein, all these drawbacks and disadvantages can be overcome. Further, the smaller size of the single domain antibody enables better tissue penetration.

In another embodiment, a method of preparing a cell composition comprising isolated CD34+ human cells is disclosed, the method comprising *in vitro* contacting a mixture of human cells, typically, derived from a subject, e.g., PBMC or bone marrow cells, with a single domain antibody of the invention and isolating cells bound by the single domain antibody. As described above, CD34+ cells can preferably be isolated from PBMC after mobilization from the bone marrow. For example, the single domain antibody can be associated with a magnetic bead for magnetic cell sorting, which is preferred, or with another detectable label, e.g., a fluorescent label for flow cytometric cell sorting. Isolated CD34+ cells can, optionally, be modified, e.g., genetically engineered. For example, they can be genetically engineered to express a recombinase. Isolated CD34+ cells or cells derived therefrom can then be formulated for transfer to a subject. They can, e.g., be transferred to the same subject from which they were isolated, for example, after a cancer treatment. They can also be transferred to another subject.

The term "a", as used herein, shall be understood to refer to "at least one". "About" shall be understood to refer to an acceptable amount of error for the quantity measured, given the nature or precision of the measurement. Typically, about refers to +/- 10% of the specified value.

All references cited herein are intended to be fully incorporated herein by reference. The following figures and examples are intended to illustrate the invention, but not to limit its scope.

| | | |
|---|---|---|
| SEQ ID NO: 1 | CDR1 of C6 and IIA12 | TFGMN |
| SEQ ID NO: 2 | CDR2 consensus sequence of C6 and IIA12 further allowing for K substitution TISAXGLRXXYXXSVXX | |
| | wherein X is any naturally occurring amino acid | |
| SEQ ID NO: 3 | CDR2 consensus sequence 1 of C6 and IIA12 | TISAX1GLRX2X3YX4X5SVKX6 |
| | wherein X1 is a polar amino acid, X2 is a polar or unpolar amino acid, X3 is a basic or polar amino acid, X4 is a polar or unpolar amino acid, X5 is a an acidic amino acid and X6 is an acidic or an unpolar amino acid. | |
| SEQ ID NO: . | 4 CDR2 consensus sequence 1 of C6 and IIA12 | TISAX1GLRX2X3YX4X5SVKX6 |
| | wherein X1 is N or T, X2 is T or V, X3 is R or N, X4 is S or A, X5 is D or E, and X6 is D or G | |
| SEQ ID NO: 5 | CDR2 of C6 | TISATGLRVNYAESVKG |
| SEQ ID NO: 6 | CDR2 of IIA12 | TISANGLRTRYSDSVKD |
| SEQ ID NO: 7 | CDR3 of C6 and IIA12 | GWPSPIY |
| SEQ ID NO: 8 C6 | | |
| EVQLVESGGGLVQPGGSLRLSCAASGLTFSTFGMNWYRQASGKERELVATISATGLRVNYAESVKGRFT VSRDNVKDTVYLQMNSLIPEDTAVYECAAGWPSPIYWGQGTRVTVSS | | |
| SEQ ID NO: 9 IIA12 | | |
| HVQLVESGGGLVQPGGSLRLSCAASGLTFSTFGMNWYRQASGKERELVATISANGLRTRYSDSVKDRF TISRDNSKDTVYLQMSSLIPEDTAVYECAAGWPSPIYWGQGAQVTVSS | | |
| SEQ ID NO: 10 IG7 | | |
| HVQLVESGGGLVQPGGSLTLSCVVSGLTFSTYGMTWYREASGKEREFVAYMSNGGLRRNYAESVKDR FTISRDNVKNTVYLQMNSLIPEDTAVYYCAAGWPSPVYWGQGTRVTVSS | | |
| SEQ ID NO: 11 IIG2 | | |
| QVQLVESGGGLVQPGGSLRLSCAASGTIFDIYDMDWYRQAPEKQRELVAGVSSDGLTNYADSVKGRFTI SRDNAKNTVYLQMNSLKPEDTAVYYCWAGNERAGPSFGSWGQGSPVTVSS | | |
| SEQ ID NO: 12 A12 | | |
| DVQLVESGGGLVQAGGSLRLSCAASGSIFSIDGMGWYRQAPGKERELVAAISNVGSTYYANSLKGRFAI SRDNAKNTVYLQMNSLKPEDTAMYYCAAYRAKTDKESDSETFAAWGQGTQVTVSS | | |
| SEQ ID NO: 13 IIC3 | | |
| HVQLVESGGGLVQAGGSLRLSCAASGTIFSISGMGWYREAPGKERELVASISSGGSTYYADSVKGRFTIS RDNAKNTLYLQMNSLNAEDTATYYCAALEGRGETFASWGQGAQVTVSS | | |
| SEQ ID NO: 14 IB11 | | |
| DVQLVESGGGLVQAGGSLRLSCAASENIFSIDAMGWYRQAPGKEREVVAVISSGGSTYYGDSVKGRFAI SRDNAKNTLFLQMNSLKPEDTAMYYCAALGGIEETFRSWGQGTQVTVSS | | |
| SEQ ID NO: 15 IH8 | | |
| QVQLVESGGGLVQPGGSLRLSCAASGFAFSSYYMHWFRQAPGKEREKVASISNGGGITHYEDSVKGRF TISRDNAKNMVYLEMNSLKPEDTAVYYCKAGTTYWGQGTPVTVSS | | |
| SEQ ID NO: 16 IG4 | | |
| HVQLVESGGGLVQPGGSLRLSCAASGFAFNMYVMRWYRQAPGKERELVATITNGGGITHYEDSVKGRF TISRDNAKNTVYLQMNSLKPEDTAVYYCQAGTTYWGQGTPVTVSS | | |
| SEQ ID NO: 17 CDR1 of IG7 | TYGMT | |
| SEQ ID NO: 18 CDR2 of IG7 | YMSNGGLRRNYAESVKD | |
| SEQ ID NO: 19 CDR3 of IG7 | GWPSPVY | |
| SEQ ID NO: 20 CDR1 of IIG2 | IYDMD | |
| SEQ ID NO: 21 CDR2 of IIG2 | GVSSDGLTNYADSVKG | |
| SEQ ID NO: 22 CDR3 of IIG2 | GNERAGPSFGS | |
| SEQ ID NO: 23 CDR1 of A12 | IDGMG | |
| SEQ ID NO: 24 CDR2 of A12 | AISNVGSTYYANSLKG | |
| SEQ ID NO: 25 CDR3 of A12 | YRAKTDKESDSETFAA | |
| SEQ ID NO: 26 CDR1 of IIC3 | ISGMG | |
| SEQ ID NO: 27 CDR2 of IIC3 | SISSGGSTYYADSVKG | |
| SEQ ID NO: 28 CDR3 of IIC3 | LEGRGETFAS | |
| SEQ ID NO: 29 CDR1 of IB11 | IDAMG | |
| SEQ ID NO: 30 CDR2 of IB11 | VISSGGSTYYGDSVKG | |
| SEQ ID NO: 31 CDR3 of IB11 | LGGIEETFRS | |
| SEQ ID NO: 32 CDR1 of IH8 | SYYMH | |
| SEQ ID NO: 33 CDR2 of IH8 | SISNGGGITHYEDSVKG | |
| SEQ ID NO: 34 CDR3 of IH8 | GTTY | |
| SEQ ID NO: 35 CDR1 of IG4 | MYVMR | |
| SEQ ID NO: 36 CDR2 of IG4 | TITNGGGITHYEDSVKG | |
| SEQ ID NO: 37 CDR3 of IG4 | GTTY | |
| SEQ ID NO: 74: TISAX1GLRX2X3YX4X5SVX6X7 | CDR2 consensus sequence of C6 and IIA12 TISAXGLRXXYXXSVKX wherein X is any naturally occurring amino acid | |
| SEQ ID NO: 75: | CDR2 consensus sequence 1 of C6 and IIA12 allowing for K to R substitution | |
| | wherein X1 is a polar amino acid, X2 is a polar or unpolar amino acid, X3 is a basic or | |
| | polar amino acid, X4 is a polar or unpolar amino acid, X5 is an acidic amino acid, X6 is K or R and X7 is an acidic or an unpolar amino acid. | |
| SEQ ID NO: 76: | CDR2 consensus sequence 2 of C6 and IIA12 allowing for K to R substitution TISAX1GLRX2X3YX4X5SVX6X7 | |
| | wherein X1 is N or T, X2 is T or V, X3 is R or N, X4 is S or A, X5 is D or E, X6 is K or R and X7 is D or G. | |
| SEQ ID NO: 77 | CDR2 of C6 allowing for K to R substitution | TISATGLRVNYAESVXG, |
| | Wherein X is R or K, optionally, R | |
| SEQ ID NO: 78 | CDR2 of IIA12 allowing for K to R substitution | TISANGLRTRYSDSVXD |
| | Wherein X is R or K, optionally, R | |
| SEQ ID NO: 79: | C6 with K to R substitutions | |
| EVQLVESGGGLVQPGGSLRLSCAASGLTFSTFGMNWYRQASGRERELVATISATGLRVNYAESVRGRF TVSRDNVRDTVYLQMNSLIPEDTAVYECAAGWPSPIYWGQGTRVTVSS | | |
| SEQ ID NO: 80: | IIA12 with K to R substitutions | |
| HVQLVESGGGLVQPGGSLRLSCAASGLTFSTFGMNWYRQASGRERELVATISANGLRTRYSDSVRDRF TISRDNSRDTVYLQMSSLIPEDTAVYECAAGWPSPIYWGQGAQVTVSSBrief description of the | | |

### Brief description of the figures

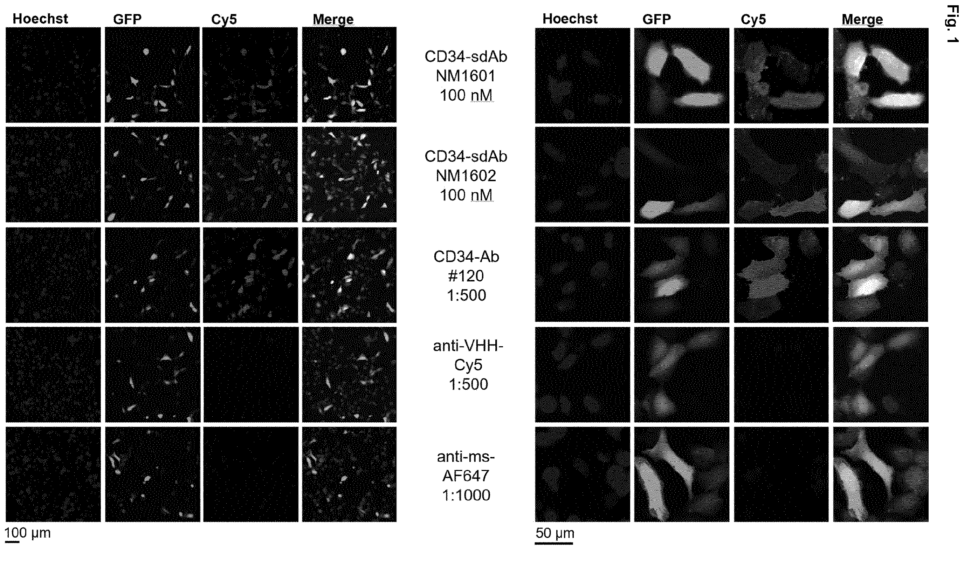

Fig. 1 Staining of live cells transiently expressing CD34. Representative pictures of U2OS cells transiently expressing GFP and CD34, stained by CD34 specific nanobodies C6 and IIA12. Detection of bound nanobodies was by Cy^{™}5 AffiniPure Goat Anti-Alpaca IgG, VHH domain (Jackson 128-175-232). Nuclei were stained with Hoechst 33342 dye. For detection of CD34, a mouse anti human CD34 antibody (biolegend 343502) was also used and detected with anti mouse AF647 secondary antibody.

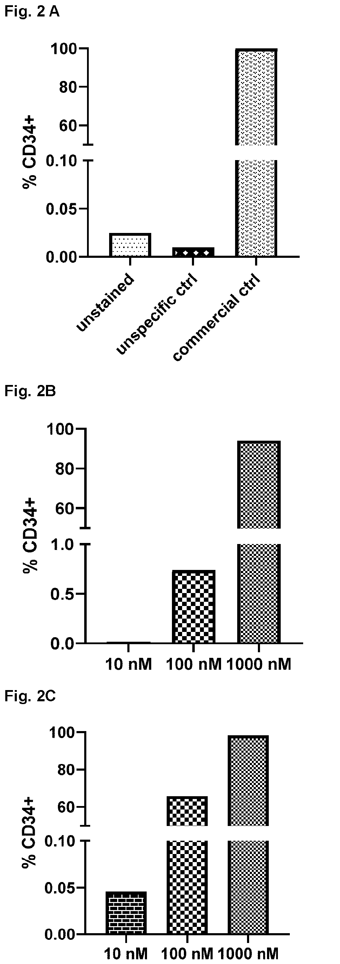

Fig. 2 Frequency of CD34 positive cells determined with different antibodies and nanobodies. A: Frequency of CD34 positive cells determined by the commercially available antibody ("commercial ctrl") in comparison to the unspecific control single domain antibody ("unspecific ctrl") and cells that were left unstained ("unstained"). B: Frequency of CD34 positive cells determined by staining with three different concentrations of the CD34-specific single domain antibody clone C6. C: Frequency of CD34 positive cells determined by staining with three different concentrations of the CD34-specific single domain antibody clone IIA12. Purified CD34 cells were first stained with a viability dye to enable exclusion of dead cells and then with the respective antibody or single domain antibody for 20 minutes at room temperature and washed once before measurement. Flow cytometry was done on a Miltenyi Biotec MACSQuant Analyzer 10.
**Fig. 3** Assessment of unspecific binding determined by staining of PBSC. Cells were stained with a viability dye to enable exclusion of dead cells and subsequently with the highest concentration (1000 nM) of the CD34-specific nanobodies in comparison to a commercially available antibody ("commercial ctrl") and an unspecific control single domain antibody ("unspecific ctrl") and cells that were left unstained ("unstained"). Staining was performed for 20 minutes at room temperature and cells were washed once before measurement. Flow cytometry was done on a Miltenyi Biotec MACSQuant Analyzer 10.
**Fig. 4** Sequences of different nanobodies identified (SEQ ID NO: 8-16) and their framework regions and CDRs.

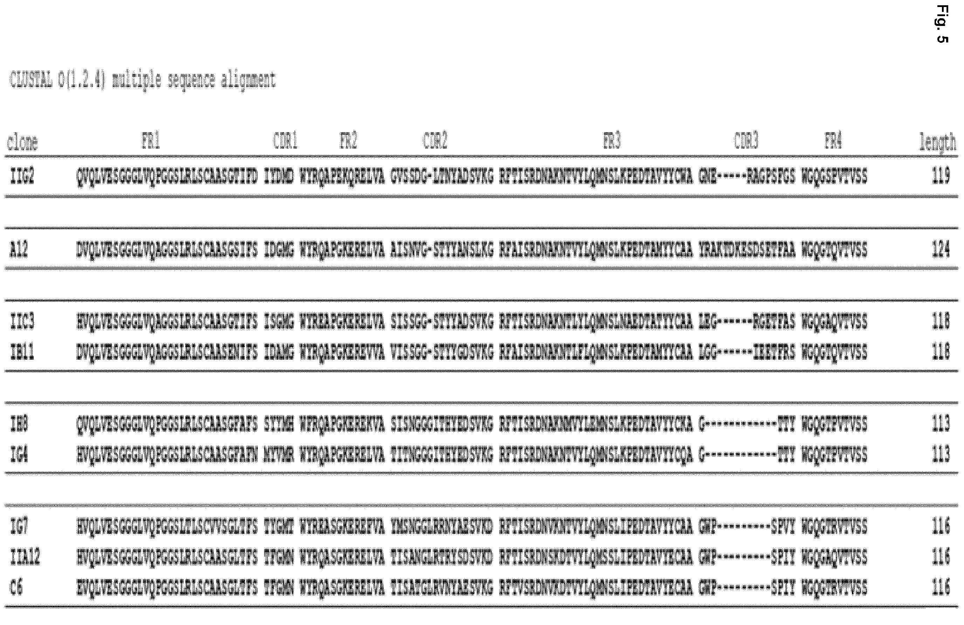

**Fig. 5** Sequence alignment of different nanobodies identified (SEQ ID NO: 8-16)

### Examples

### Example 1: Generation of CD34-specific nanobodies

For generation of CD34-specific nanobodies, in summary, human CD34 was produced in HEK293 cells, the degree of glycosylation of the protein was reduced enzymatically, and the protein was then used in a mixture with untreated protein for immunization of an alpaca *(Vicugna paces).* Success of immunization was shown by seroconversion. From a blood sample of the immunized animal, the genetic repertoire of the generated nanobodies could successfully be transferred into a single domain antibody phage library. Based on this, with phage display, anti-CD34 nanobodies were selected and specific binding proven in a phage-ELISA. By sequence analysis, nine nanobodies from five diverse sequence clusters could be identified. For validation, the identified nanobodies were expressed and validated by staining of living cells transiently expressing CD34. For nanobodies C6 and IIA12, binding to CD34 in the cellular context was confirmed.

### 1.1 Preparation of human CD34 for immunization

The extracellular part of human CD34 comprises several posttranslational modifications (PTMs). Based on these, the part can be subdivided into a hypermodified non-folded mucin domain and a cysteine-containing globular domain. For the expression of the extracellular domain (AA32-290 of human CD34 (UniProt ID - P28906), an expression plasmid with N-terminal signal peptide (AA1-31) and C-terminal His tag was generated. The protein produced in transiently transfected HEK293 suspension cells was purified from cell free culture supernatant with immobilized metal ion affinity chromatography (IMAC) and analyzed. The theoretical molecular weight of the expressed protein is 29 kDa. Because of the high degree of glycosylation, the reduced protein can be detected in gels in two diffuse bands at about 55 and mainly about 100 kDa. To increase accessibility of the core protein to nanobodies, the degree of glycosylation of the protein was significantly reduced enzymatically. For immunization and selection of CD34-specific nanobodies, a mixture of untreated and partly deglycosylated protein was employed.

Cellular expression of full length membrane-bound human CD34 (UniProtKB P28906) was cloned into the expression vector pCDNA IRES GFP. Due to an internal ribosomal entry site (IRES) in the mRNA sequence, GFP is co-expressed from the same transcript.

### 1.2 Immunization

An alpaca *(Vicugna paces)* was immunized according to known protocols.

The immune response of the animal was tested by a serum ELISA. Dilutions of serum were incubated with immobilized CD34. To show seroconversion, bound antibodies were detected with an HRP-conjugated anti VHH antibody (Jackson ImmunoResearch - Peroxidase AffiniPure^{™} Goat Anti-Alpaca IgG, VHH domain) and quantified after conversion of substrate by determining absorbance at 450nm. In dilutions 10⁻¹ and 10⁻⁵, compared to control (serum before immunization), a significantly increased amount of antibodies could be detected, proving a successful immunization.

After seroconversion, PBMC were isolated from a fresh blood sample. 2.5×10⁷ cells were used for the following steps.

### 1.3 Preparation of an anti-CD34 single domain antibody phage library

From the isolated PBMCs, mRNA was extracted and transcribed to cDNA. Based on the cDNA, by amplification and subsequent ligation of adapters, the complete single domain antibody genetic repertoire of the sample was cloned into a phagemid vector. For preparation of a single domain antibody phage library, in total 1×10⁷ E. coli were transformed and a continuous glycerol culture started.

### 1.4 Selection of CD34-specific nanobodies

### 1.4.1 Solid-phase Phage Display (Panning on immobilized CD34 antigen)

For selection of CD34-specific nanobodies, 2 cycles of solid-phase phage display were carried out. On immobilized CD34, compared to the control, specific phages were 31 fold concentrated at first. In the second cycle, another 7.6×10³-fold enrichment was obtained.

### 1.4.2 Identification and sequence analysis of CD34-specific nanobodies

Binding characteristics of 300 randomly selected single clones were tested by a phage ELISA. The single domain antibody sequences of the phages selectively binding CD34 were determined by sequencing. From CD34 positive Phages, 9 single domain antibody sequences from 5 different sequence clusters were identified.

### 2.4.3 Validation of selected nanobodies on the protein level

The identified CD34 nanobodies were expressed in small scale in E. *coli.* Expressed nanobodies were detected with SDS-PAGE of periplasmatic extracts and Coomassie staining or Western Blot. To validate binding to CD34 in cellular context, live U2OS cells transiently expressing CD34 were stained with periplasmatic extracts. Specific binding was shown for nanobodies C6, IIA12 and, more weakly, for IG7 (data not shown). The staining was repeated with purified nanobodies.

### 1.4.3 Expression and chromatographic purification of nanobodies

To validate specific CD34 binding on cells, nanobodies were expressed in 1L expression culture and purified by affinity chromatography (IMAC) and size exclusion chromatography (SEC) Staining of live cells C6 und IIA12 is shown in Fig. 1.

### Example 2: Binding of CD34-specific nanobodies to human CD34+ cells

Two different clones of CD34-specific nanobodies, C6 and IIA12, were each coupled to the fluorescent dye ATTO 647. As a negative control, an unspecific single domain antibody also coupled to ATTO 647 was provided. As a positive control, a validated commercially available anti-CD34 antibody was used at the concentration recommended by the vendor (BD Pharmingen^{™} PE-Cy^{™}7 Mouse AntiHuman CD34, catalogue number 560710).

Two different types of human primary cells were used for the experiments: human peripheral blood with mobilized hematopoietic stem cells (Hopman RK, DiPersio JF. Advances in stem cell mobilization. Blood Rev. 2014 Jan;28(1):31-40. doi: 10.1016/j.blre.2014.01.001), which only contain a small fraction of CD34+ cells, subsequently described as peripheral blood stem cells (PBSC). From this blood product, CD34 positive cells were isolated using magnetically labelled anti-CD34 antibodies, which bind to the target cells and then to an external magnet which allows for decanting of any unwanted cells while the cells of interest remain in the reagent tube (https://www.stemcell.com/easysep-human-cd34-positive-selection-kit-ii.html; Kaur M, et al.. Induction and Therapeutic Targeting of Human NPM1c+ Myeloid Leukemia in the Presence of Autologous Immune System in Mice. J Immunol. 2019 Mar 15;202(6):1885-1894. doi: 10.4049/jimmunol.1800366).

The PBSC were used to determine any unspecific binding of the clones to CD34 negative cells. *In vitro* experiments were conducted with three different concentrations of the nanobodies were used.

First, control stainings were conducted with the commercially available anti-CD34 antibody and the unspecific control single domain antibody (1000 nM) on purified CD34 cells; the results are displayed in Figure 1A. As expected, nearly all CD34 positive cells could be detected by the commercially available anti-CD34 antibody. The unspecific control single domain antibody displayed a very low background (i.e. less than 1 % of the cells were stained).

Next, different concentrations of the CD34-specific nanobodies were tested. Fig. 1B shows the results of CD34+ cells stained with the anti-CD34 single domain antibody clone C6 at three different concentrations (10 nM, 100 nM and 1000 nM). At a concentration of 1000 nM, staining with the clone C6 resulted in a robust detection of the CD34 positive cells. Also, a strong concentration-dependent decrease of the proportion of detected cells could be observed.

The same concentrations were tested with the CD34-specific single domain antibody clone IIA12; the results are displayed in Fig. 1C. Comparable to clone C6, staining with the clone IIA12 at a concentration of 1000 nM resulted in a good detection of the CD34 positive cells. Also, a concentration-dependent decrease of the proportion of detected cells could be observed.

In a final step, the specific nanobodies as well as the controls were tested on PBSC, which only contain a small fraction of CD34 positive cells to check for unspecific binding. The results are displayed in Fig. 2. The frequency of CD34 positive cells determined with the CD34-specific nanobodies corresponds to the expected frequency (< 2 %, Huntsman HD, et al., Blood. 2015) of CD34 positive cells within PBSC. It is also comparable to the frequency that could be determined with the commercially available antibody.

In summary, the experiments demonstrate a concentration-dependent specificity of the two tested CD34-specific single domain antibody clones which also displayed very low levels of unspecific binding on a mixture of cells that were mainly CD34 negative (PBSC). The unspecific control antibody that was provided with the same labelling as the specific clones did not show any significant binding to either CD34 positive cells or PBSC.

### Reference list

WO 2002/44409
WO 2008/083931
WO 2011/147590
WO 2016/034553 A1
Huntsman HD, et al., Human hematopoietic stem cells from mobilized peripheral blood can be purified based on CD49f integrin expression. Blood 2015; 126 (13): 1631-1633. doi: 10.1182/blood-2015-07-660670
Hopman RK, DiPersio JF. Advances in stem cell mobilization. Blood Rev. 2014 Jan;28(1):31-40. doi: 10.1016/j.blre.2014.01.001
Kaur M, et al.. Induction and Therapeutic Targeting of Human NPM1c+ Myeloid Leukemia in the Presence of Autologous Immune System in Mice. J Immunol. 2019 Mar 15;202(6):1885-1894. doi: 10.4049/jimmunol. 1800366)

## Claims

1. A nucleic acid encoding a single domain antibody capable of specifically binding to human CD34, the single domain antibody comprising a CDR1 having at least 60%, preferably, at least 80% sequence identity to SEQ ID NO: 1, a CDR2 having at least 80% sequence identity to SEQ ID NO: 2, and a CDR3 having at least 85% sequence identity to SEQ ID NO: 7.

2. The nucleic acid of claim 1, wherein
a) the CDR1 has SEQ ID NO: 1, the CDR2 has SEQ ID NO: 2, and the CDR3 has SEQ ID NO: 7, wherein, optionally, the CDR2 has SEQ ID NO: 74, or
b) the CDR1 has SEQ ID NO: 17, the CDR2 has SEQ ID NO: 18, and the CDR3 has SEQ ID NO: 19.

3. The nucleic acid of claim 2, wherein the CDR1 has SEQ ID NO: 1, the CDR3 has SEQ ID NO: 7 and the CDR2 has SEQ ID NO: 75, wherein preferably, the CDR2 has SEQ ID NO: 76, optionally, SEQ ID NO: 4.

4. The nucleic acid of claim 3, wherein the CDR2 has SEQ ID NO: 78, preferably, SEQ ID NO: 6, wherein optionally, the single domain antibody has at least 90%, e.g., 100% sequence identity to SEQ ID NO: 9.

5. The nucleic acid of claim 3, wherein the CDR2 has SEQ ID NO: 77, preferably, SEQ ID NO: 5, wherein optionally, the single domain antibody has at least 90%, e.g., 100% sequence identity to SEQ ID NO: 8.

6. The nucleic acid of any of claims 1-5, which is an expression construct wherein the sequence encoding the single domain antibody is functionally linked to a promotor, wherein, optionally, the sequence linked to the single domain antibody is further linked to a coding sequence encoding a fusion partner configured so that the single domain antibody is expressed as a fusion protein with the fusion partner.

7. A single domain antibody encoded by the nucleic acid of any of the preceding claims.

8. The single domain antibody of claim 7, wherein the single domain antibody is associated with a moiety selected from the group comprising
a) a transfer moiety selected from the group comprising a lipid nanoparticle (LNP), a viral particle, a liposome, an exosome or a bacterial minicell,
b) a drug
c) a detectable label selected from the group comprising a fluorophore, a radionuclide, a magnetic particle or a nucleic acid and/or
d) a moiety increasing stability and/or serum half-life selected from the group comprising polyethylene glycole or hydroxyethyl starch,
wherein, preferably, the single domain antibody is associated with a transfer moiety, optionally, an LNP.

9. The single domain antibody of claim 8, wherein the single domain antibody is associated with a transfer moiety comprising an active agent selected from the group comprising a nucleic acid, a drug or a detectable label, preferably, a nucleic acid such as mRNA.

10. A host cell comprising the nucleic acid of any of claims 1-6, optionally, expressing the single domain antibody.

11. A pharmaceutical composition comprising the nucleic acid of any of claims 1-6 or the single domain antibody of any of claims 7-9 or the host cell of claim 10,
wherein, optionally, the composition further comprises at least one pharmaceutically acceptable excipient and/or solvent.

12. Use of the single domain antibody of any of claims 7-9 associated with a moiety
for targeting the moiety to a cell expressing human CD34, wherein said cell optionally is a human hematopoietic stem cell, e.g., in vitro.

13. The pharmaceutical composition of claim 11 comprising a single domain antibody associated with a moiety, for use in targeting the moiety to a cell expressing human CD34,
wherein said cell optionally is a human hematopoietic stem cell,
preferably, for use in transfecting a cell expressing human CD34 with a nucleic acid, wherein the moiety is a transfer moiety comprising a nucleic acid.

14. A method of preparing a genetically engineered human cell expressing CD34, optionally, a genetically engineered human hematopoietic stem cell, the method comprising
a) *in vitro* contacting a cell expressing human CD34 with a single domain antibody of any of claims 8 or 9, or
b) administering to a human subject in need thereof a pharmaceutical composition of claim 11 comprising a single domain antibody associated with a moiety,
wherein the moiety is a transfer moiety comprising a nucleic acid.

15. A method for detecting a cell expressing human CD34, the method comprising
a) *in vitro* contacting a human cell expressing CD34 with a single domain antibody of any of claims 8 or 9 associated with a detectable label, wherein the cell is optionally comprised in a mixture of different cells or in a tissue or organ, or
b) administering to a human subject a single domain antibody of any of claims 8 or 9 associated with a detectable label,
and determining binding of said single domain antibody to the cell.

16. A method of preparing a cell composition comprising isolated CD34+ human cells, the method comprising *in vitro* contacting a mixture of human cells derived from a subject, selected from the group comprising PBMC or bone marrow cells, with a single domain antibody of any of claims 7-8 and isolating cells bound by the single domain antibody.
